Europäisches Patentamt

European Patent Office

Office européen des brevets

(11) Publication number: **0 418 582 A2**

# EUROPEAN PATENT APPLICATION

(21) Application number: **90116153.9**

(51) Int. Cl.5: **A61K 31/40, A61K 31/675**

(22) Date of filing: **23.08.90**

The title of the invention has been amended (Guidelines for Examination in the EPO, A-III, 7.3).

(30) Priority: **31.08.89 US 401377**

(43) Date of publication of application:
**27.03.91 Bulletin 91/13**

(84) Designated Contracting States:
**AT BE CH DE DK ES FR GB GR IT LI LU NL SE**

(71) Applicant: **E.R. SQUIBB & SONS, INC.**
**P.O.Box 4000**
**Princeton New Jersey 08543-4000(US)**

(72) Inventor: **Moore, Luana R.C.**
**RD No.1, Box 447**
**Pipersville, PA 18947(US)**

(74) Representative: **Josif, Albert et al**
**Baaderstrasse 3**
**W-8000 München 5(DE)**

(54) **Method for treating hypermotility diseases of the bowel employing an ace inhibitor.**

(57) A method is provided for treating hypermotility diseases of the bowel, such as irritable bowel syndrome (IBS) or other inflammatory bowel diseases as well as chronic diarrhea states using an angiotensin converting enzyme inhibitor such as captopril, fosinopril, ceranapril, enalapril or lisinopril which may be administered by suppository, enema or by oral dosage forms that release drug in the colon. A new suppository formulation for use in such method is also provided.

EP 0 418 582 A2

# METHOD FOR TREATING HYPERMOTILITY DISEASES OF THE BOWEL EMPLOYING AN ACE INHIBITOR AND FORMULATION FOR USE THEREIN

The present invention relates to a method for treating hypermotility diseases of the bowel, such as irritable bowel syndrome (IBS), other inflammatory bowel diseases as well as chronic diarrhea, using an angiotensin converting enzyme (ACE) inhibitor, and to a suppository formulation for use therein.

In accordance with the present invention, a method is provided for treating hypermotility diseases of the bowel, such as irritable bowel syndrome (IBS) or other inflammatory bowel diseases as well as chronic diarrhea states, in mammalian species, wherein a therapeutically effective amount of an angiotensin converting enzyme inhibitor is administered systemically, such as orally or parenterally, or by enema, suppository or oral dosage forms that release ACE inhibitors in the colon.

A new suppository formulation containing an angiotensin converting enzyme inhibitor and a suppository base for use in the above method is also provided.

The term "hypermotility diseases of the bowel" will include irritable bowel syndrome (IBS), diabetic gastroenteropathy, Crohn's Disease and colitis.

The method of the invention should be carried out in conjunction with and as an adjunct to a proper nutritional diet and/or physician supervised diet to achieve the desired results.

The angiotensin converting enzyme inhibitor which may be employed herein includes substituted proline derivatives, such as any of those disclosed in U. S. Patent No. 4,046,889 or 4,105,776 to Ondetti et al mentioned above, with captopril, that is, 1-[(2S)-3-mercapto-2-methylpropionyl]-L-proline, being preferred, carboxyalkyl dipeptide derivatives, such as any of those disclosed in U.S. patent No. 4,374,829 mentioned above, with N-(1-ethoxycarbonyl-3-phenylpropyl)-L-alanyl-L-proline, that is, enalapril, being preferred.

Other examples of angiotensin converting enzyme inhibitors suitable for use herein include any of the phosphonate substituted amino or imino acids or salts disclosed in U. S. Patent No. 4,452,790 with (S)-1-[6-amino-2-[[hydroxy-(4-phenylbutyl)phosphinyl]oxy]-1-oxohexyl]-L-proline (SQ 29,852 or ceranapril) being preferred, phosphinylalkanoyl prolines disclosed in U. S. Patent No. 4,168,267 mentioned above with fosinopril being preferred, mercaptoacyl derivatives of substituted prolines, disclosed in U. S. Patent No. 4,316,906 with zofenopril being preferred, any of the phosphinylalkanoyl substituted prolines disclosed in U. S. Patent No. 4,337,201 discussed above, and the phosphonamidates disclosed in U. S. Patent No. 4,432,971 discussed above.

Other examples of ACE inhibitors that may be employed herein include Beecham's BRL 36,378 as disclosed in European patent Nos. 80822 and 60668; Chugai's MC-838 disclosed in CA. 102:72588v and Jap. J. Pharmacol. 40:373 (1986); Ciba-Geigy's CGS 14824 (3-([1-ethoxycarbonyl-3-phenyl-(1S)-propyl]-amino)-2,3,4,5-tetrahydro-2-oxo-1-(3S)-benzazepine-1 acetic acid HCl) disclosed in U.K. Patent No. 2103614 and CGS 16,617 (3(S)-[[(1S)-5-amino-1-carboxypentyl]amino]-2,3,4,5-tetrahydro-2-oxo-1H-1-benzazepine-1-ethanoic acid) disclosed in U. S. Patent No. 4,473,575; cetapril (alacepril, Dainippon) disclosed in Eur. Therap. Res. 39:671 (1986); 40:543 (1986); ramipril (Hoechst) disclosed in Eur. Patent No. 79-022 and Curr. Ther. Res. 40:74 (1986); Ru 44570 (Hoechst) disclosed in Arzneimittelforschung 35:1254 (1985), cilazapril (Hoffman-LaRoche) disclosed in J. Cardiovasc. Pharmacol. 9:39 (1987); $R_o$ 31-2201 (Hoffman-LaRoche) disclosed in FEBS Lett. 165:201 (1984); lisinopril (Merck) disclosed in Curr. Therap. Res. 37:342 (1985) and Eur. patent appl. No. 12-401, indalapril (delapril) disclosed in U. S. Patent No. 4,385,051; rentiapril (fentiapril, Santen) disclosed in Clin. Exp. Pharmacol. Physiol. 10:131 (1983); indolapril (Schering) disclosed in J. Cardiovasc. Pharmacol. 5:643, 655 (1983); spirapril (Schering) disclosed in Acta. Pharmacol. Toxicol. 59 (Supp. 5):173 (1986); perindopril (Servier) disclosed in Eur. J. Clin. Pharmacol. 31:519 (1987); quinapril (Warner-Lambert) disclosed in U. S. Patent No. 4,344,949 and CI 925 (Warner-Lambert) ([3S-[2[R(*)R(*)]]3R-(*)]-2-[2-[[1-(ethoxycarbonyl)-3-phenylpropyl]amino[-1-oxopropyl]-1,2,3,4-tetrahydro-6,7-dimethoxy-3-isoquinolinecarboxylic acid HCl) disclosed in Pharmacologist 26:243, 266 (1984), WY-44221 (Wyeth) disclosed in J. Med. Chem. 26:394 (1983).

Preferred are those ACE inhibitors which are proline or substituted proline derivatives.

The above-mentioned U.S. patents are incorporated herein by reference.

In carrying out the method of the present invention, the angiotensin converting enzyme inhibitor may be administered to mammalian species, such as horses, cattle, dogs, cats, rats and humans, and as such may be incorporated in a conventional systemic dosage form, such as a tablet, capsule, elixir or injectable, as well as enema and suppository dosage forms, and oral dosage forms that release ACE inhibitor in the colon. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms such as intramuscular, intraperitoneal, or intravenous enema and suppository forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing the ACE inhibitor in an amount within the range of from about 0.01 mg/kg to about 100 mg/kg and preferably from about 0.1 mg/kg to about 25 mg/kg.

A preferred oral dosage form , such as tablets or capsules, will contain the ACE inhibitor in an amount of from about 0.1 to about 500 mg, preferably from about 5 to about 200 mg, and more preferably from about 10 to about 150 mg.

For parenteral administration, the ACE inhibitor will be employed in an amount within the range of from about 0.005 mg/kg to about 10 mg/kg and preferably from about 0.01 mg/kg to about 1 mg/kg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 50 to 700 mg in total weight, containing the active substance in the range described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending the active substance in a conventional liquid vehicle acceptable for pharmaceutical administration so as to provide the desired dosage in one to four teaspoonfuls.

Such dosage forms can be administered to the patient on a regimen of one to four doses per day.

Enema formulations can also be prepared by dissolving or suspending the active substance in a conventional enema liquid vehicle acceptable for pharmaceutical administration so as to provide the desired dosage in one to four enemas per day.

Suppository formulations containing from about 5 to about 250 mg ACE inhibitor may be prepared as well using a conventional suppository base (such as disclosed in U.S. Patent Nos. 4,344,968, 4,265,875, and 4,542,020) so as provide the desired dosage in one to four suppositories per day.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Many of the active substances described above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as it is necessary to treat the hypermotility disorder. Sustained release forms of such formulations which may provide such amounts biweekly, weekly, monthly and the like may also be employed. A dosing period of at least one to two weeks are required to achieve minimal benefit.

The following Examples represent preferred embodiments of the present invention.

## Example 1

A captopril formulation suitable for oral administration in treating irritable bowel syndrome is set out below.

1000 tablets each containing 100 mg of 1-[(2S)-3-mercapto-2-methylpropionyl]-L-proline were produced from the following ingredients.

| 1-[(2S)-3-Mercapto-2-methylpropionyl]-L-proline (captopril) | 100 g |
|---|---|
| Corn starch | 50 g |
| Gelatin | 7.5 g |
| Avicel (microcrystalline cellulose) | 25 g |
| Magnesium stearate | 2.5 g |

The captopril and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for treating irritable bowel syndrome.

Example 2

1000 tablets each containing 200 mg of captopril are produced from the following ingredients:

| Captopril | 200 g |
|---|---|
| Lactose | 100 g |
| Avicel | 150 g |
| Corn starch | 50 g |
| Magnesium stearate | 5 g |

The captopril, lactose and Avicel are admixed, then blended with the corn starch. Magnesium stearate is added. The dry mixture is compressed in a tablet press to form 1000 505 mg tablets each containing 200 mg of active ingredient. The tablets are coated with a solution of Methocel E 15 (methyl cellulose) including as a color a lake containing yellow #6. The resulting tablets are useful in treating colitis.

Example 3

Two piece #1 gelatin capsules each containing 250 mg of captopril are filled with a mixture of the following ingredients:

| Captopril | 250 mg |
|---|---|
| Magnesium stearate | 7 mg |
| USP lactose | 193 mg. |

The resulting capsules are useful in treating irritable bowel syndrome.

Example 4

An injectable solution for use in treating irritable bowel syndrome is produced as follows:

| Captopril | 500 mg |
|---|---|
| Methyl paraben | 5 mg |
| Propyl paraben | 1 mg |
| Sodium chloride | 25 g |
| Water for injection qs. | 5 l. |

The captopril, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

Example 5 to 8

Dosage forms for use in treating irritable bowel syndrome are prepared as described in Examples 1 to 4 except that N-(1-ethoxycarbonyl-3-phenylpropyl)-L-alanyl-L-proline (enalapril) is used in place of captopril.

Example 9 and 10

A suppository formulation containing conventional suppository base such as any of those disclosed in U.S. Patent Nos. 4,344,968, 4,265,875 or 4,542,020, and N-(1-ethoxy-carbonyl-3-phenylpropyl)-L-alanyl-L-proline (40 mg), (enalapril) or captopril (25 mg), is prepared and is used to treat irritable bowel syndrome.

Example 11

A zofenopril formulation suitable for oral administration in treating irritable bowel syndrome is set out below.

1000 tablets each containing 100 mg of zofenopril are produced from the following ingredients.

| [1(S),4(S)]-1-[3-(benzoylthio)-2-methyl-1-oxopropyl-4-(phenylthio)-L-proline (zofenopril) | 100 g |
|---|---|
| Corn starch | 50 g |
| Gelatin | 7.5 g |
| Avicel (microcrystalline cellulose) | 25 g |
| Magnesium stearate | 2.5 g |

The zofenopril and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for treating irritable bowel syndrome.

Example 12

A modified release beadlet formulation capable of slowly releasing the angiotensin converting enzyme inhibitor captopril over a period of up to 6 hours and having the following composition was prepared as described below.

5

| Ingredient | Amount in Parts by Weight |
|---|---|
| Captopril | 27 |
| Citric aicd | 30 |
| Microcrystalline cellulose* | 43 |

*amount may vary to reflect chemical purity of captopril

The above ingredients were mixed and kneaded using water in a planetary mixer to form a wet mass. The wet mass was passed through a Nica E140 extruder to form an extrudate (~1 mm diameter). The extrudate was then passed through a Nica spheronizer to form beadlets. The beadlets were then dried at 40°C for 12-18 hours in a tray drying oven or for 2-4 hours in a fluid bed dryer. A fraction of the so-formed beadlets were filled into hard shell pharmaceutical capsules for use in treating irritable bowel syndrome.

Example 13

A modified release coated-beadlet formulation having the following composition was prepared as follows.

| (i) | Core | | mg/dose |
|---|---|---|---|
| | Captopril | | 100 mg |
| | Microcrystalline cellulose | | 159.1 mg |
| | Citric acid | | 37. mg |
| | Lactose | | 74.1 mg |
| (ii) | Sealcoat | | |
| | Hydroxypropyl methyl cellulose | ca. | 8.3 mg |
| | Polyethylene glycol | ca. | 2.8 mg |
| (iii) | Barriercoat | | |
| | Cellulose acetate phthalate | ca. | 4.2 mg |
| | Acetylated monoglycerides (Myvacet®9-40) | ca. | 1.3 mg |

The beadlet cores were prepared as described in Example 12. After the dried beadlets were formed, they were coated via a two step process as follows. An aqueous solution of hydroxypropyl methyl cellulose (7.5% by weight) and polyethylene glycol (2.5% by weight) was prepared and sprayed on to the beadlets to form a sealcoat. The beadlets were then coated with a barriercoat using an aqueous dispersion of cellulose acetate phthalate (30% by weight) mixed with acetylated monoglycerides (9.5% by weight). The beadlets were then filled into hard shell pharmaceutical capsules which are useful in treating chronic diarrhea.

Example 14

A modified release coated-beadlet formulation having the following composition was prepared as follows.

| Ingredient | | % by Weight of Coated Beadlet |
|---|---|---|
| Core | | |
| Captopril | | 26.2 |
| Citric acid | | 29.1 |
| Microcrystalline cellulose | | 41.8 |
| Film coating | | |
| Hydroxypropylmethyl cellulose phthalate | ca. | 2.6 |
| triethyl citrate | ca. | 0.3 |

The beadlet cores were prepared as described in Example 12.

Hydroxypropylmethyl cellulose phthalate (9 parts) and triethylcitrate (1 part) were dissolved in ethyl alcohol (90 parts) and then sprayed on to the beadlets to form coated product. The so-formed beadlets were then filled into hard shell pharmaceutical capsules which are useful in treating colitis or Crohn's disease.


Examples 15 to 19


Following the procedure of Examples 13 to 15 except substituting the following ACE inhibitor, organic acid and binder-excipients, the following beadlet compositions may be prepared which are useful in treating irritable bowel syndrome.

| Ex. No. | ACE Inhibitor | Organic acid | Binder |
|---|---|---|---|
| 15. | N-(1-ethoxycarbonyl-3-phenyl-propyl)-L-proline | Citric acid | Microcrystalline cellulose |
| 16. | (S)-1-[6-Amino-2-[[hydroxy(4-phenylbutyl)phosphinyl]-oxy]-1-oxohexyl]-L-proline | Malic acid | Microcrystalline cellulose and hydroxypropyl methyl cellulose |
| 17. | Lisinopril | Tartaric acid | Na carboxymethyl cellulose |
| 18. | Zofenopril | Succinic acid | Gelatin, pectin and Na carboxymethyl cellulose |
| 19. | Fosinopril | Maleic acid | Microcrystalline cellulose |

EP 0 418 582 A2

**Claims**

1. Use of an angiotensin converting enzyme inhibitor for preparing a drug useful for treating hypermotility diseases of the bowel, in a mammalian specie.

2. An angiotensin converting enzyme inhibitor according to claim 1, characterized in that it is a phosphonate substituted amino or imino acid or salt thereof, a proline derivative, a substituted proline derivative, a mercaptoacyl derivative of a substituted proline, a carboxyalkyl dipeptide derivative, a phosphinylalkanoyl proline derivative or a phosphonamidate derivative.

3. An angiotensin converting enzyme inhibitor as claimed in claim 1, characterized in that it is a proline derivative or a substituted proline derivative.

4. An angiotensin converting enzyme inhibitor according to claim 1, which is a carboxyalkyl dipeptide derivative.

5. An angiotensin converting enzyme inhibitor according to claim 1, which is a phosphinylalkanoyl proline derivative, a phosphoramidate derivative, or a phosphonate substituted amino or imino acid or salt thereof.

6. An angiotensin converting enzyme inhibitor according to claim 1, which is captopril.

7. An angiotensin converting enzyme inhibitor according to claim 1, which is enalapril.

8. An angiotensin converting enzyme inhibitor according to claim 1, which is lisinopril.

9. An angiotensin converting enzyme inhibitor according to claim 1, which is zofenopril.

10. An angiotensin converting enzyme inhibitor according to claim 1, which is fosinopril.

11. An angiotensin converting enzyme inhibitor according to claim 1, which is (S)-1-[6-amino-2-[[hydroxy(4-phenyl-butyl)phosphinyl]oxy]-1-oxohexyl]-L-proline (ceranapril).

12. An angiotensin converting enzyme inhibitor according to claim 1, characterized in that for its administration it is administered in single or divided doses of from about 0.1 to about 500 mg/one to four times daily.

13. Use of the inhibitor according to claim 1, for treating irritable bowel syndrome.

14. Use of the inhibitor according to claim 1, for treating Crohn's Disease or colitis.

15. Use of the inhibitor according to claim 1, for treating chronic diarrhea.

16. Use of the inhibitor according to claim 1, for treating diabetic gastroenteropathy.

17. Use of an angiotensin converting enzyme inhibitor according to claim 1, including its administering in the form of a suppository.

18. Use of the ACE inhibitor according to claim 1, including its administering in the form of an enema.

19. Use of an angiotensin converting enzyme inhibitor for preparing a drug useful for treating irritable bowel syndrome mammalian, in a mammalian specie.

20. The inhibitor as defined in claim 19, wherein the angiotensin converting enzyme inhibitor administered is captopril, fosinopril, ceranapril, zofenopril, enalapril or lisinopril.

21. A suppository formulation comprising an angiotensin converting enzyme inhibitor and a suppository base carrier therefor.

22. The formulation as defined in claim 21, wherein the angiotensin converting enzyme inhibitor is captopril, zofenopril, fosinopril, ceranapril, enalapril or lisinopril.